# EUROPEAN PATENT APPLICATION

(11) **EP 2 153 772 A1**
(43) Date of publication of application: **17.02.2010**
(21) Application number: 08014407.4
(22) Date of filing: 13.08.2008
(51) Int. Cl.: A61B 5/00, G01N 21/47

(54) **Method for determining optical and spatial characteristics of an inclusion in a turbid medium using multiple-scattering optical tomography**

(71) Applicant: Siemens Aktiengesellschaft, 80333 München (DE)
(72) Inventor: Latchezar, Avramov, 1113 Sofia (BG); Bliznakova, Irina, 1225 Sofia (BG); Borisova, Ekaterina, 1387 Sofia (BG); Dreischuh, Tanja, 1000 Sofia (BG); Gurdev, Ljuan, 1404 Sofia (BG); Stoyanov, Ditmar, 1172 Sofia (BG); Orlin Ivanov Vankov, BG-1164 Sofia (BG)
(74) Representative: Fischer, Michael

(57) **Abstract**

Method for determining optical and spatial characteristics of an inclusion in a turbid medium using multiple-scattering optical tomography, comprising the steps of emitting a collimated laser incident radiation along an optical axis, originating from a continuous wave laser situated in a predefined position whereby the incident radiation is scattered immediately after entering the turbid medium through a contact surface, the scattering yielding a totality of backscattered secondary photon beams, spatially filtering the totality of backscattered secondary photon beams, the filtering yielding a number of specific non-overlapped back scattered photon beams, wherein said specific photon beams have a substantially parallel orientation to the optical axis, determining a spatial distribution of an optical output power of the medium comprising the inclusion, computing the spatial characteristics of the inclusion, determining a contrast and a sign of the said contrast of the inclusion and computing the optical characteristics of the inclusion and of the turbid medium.

## Description

The invention relates to a method for determining optical and spatial characteristics of an inclusion in a turbid medium using multiple-scattering optical tomography, especially by using a continuous wave laser.

Imaging through turbid media has become an important field of research, for its great potential in diverse fields of applications like meteorology, ecology, aviation, astrophysics, etc. Currently, emphasis has mostly been laid on medical applications using infrared or visible light instead of harmful X-rays for imaging through tissues. This is referred to as optical tomography. Studies have shown that the absorption and scattering characteristics at a given wavelength by normal tissues differ significantly from the characteristics of other tissues like tumors, kidney stones, etc. This behavior has been adapted for discriminating or locating tumors. Basic limitations are posed by the effect of multiple scattering of light as well as hardware schematics whereby minimizing the multiple scattering effects are difficult to realize.
Most of well known optical tomography techniques, like the double-side optical tomography, are based mainly on a transmission approach for detection of radiation transmitted through the turbid medium, in other words based on a forward scattering method. This method is characterized by double-side schematics, problems with a determination of the optical characteristics of the turbid medium, large measurement times of ballistic and snake photons and an impossibility to distinguish between normal and abnormal tissues.
Other methods for biomedical imaging are using a frequency-domain approach as it is simpler in terms of hardware and feature single sided schematics, however they are characterized by small sounding depths, a very low level of useful signal and a low spatial resolution.
All present solutions are extremely complicated and expensive, using as a rule more than a single high-technology laser source, precise electronics for acquisition and imaging of information, consecutively requiring time-consuming calibration procedures. Moreover, they present ambiguous solutions of corresponding inverse mathematical problems involving the multiple scattering effects.

A turbid medium is a strongly scattering material, for example a tissue. An inclusion is a volume of a different material as the turbid medium enclosed in the strongly scattering material.
Spatial characteristics are to be understood as a totality of information comprising a position of the inclusion relative to a reference point, as well as 3 dimensional coordinates describing a volume of the inclusion.
Optical characteristics describe an interaction of light with the turbid medium and the inclusion, like an absorption coefficient µa, an integral scattering coefficient µs, a back scattering coefficient β, an extinction coefficient e being a sum of the absorption coefficient and the integral scattering coefficient, and a scattering angular dependence χ(θ) on the angle θ between an incident and a scattering direction, which is assumed to be sharply forward oriented, meaning that it sharply drops to zero at an angle close to zero.

In this document, the term forward scattering is used for photons which have undergone few scattering events and travel in the direction of the incident radiation under certain angles, less than π/2 to the incident radiation direction.
The term back scattering, on the contrary, refers to photons which have undergone multiple scattering events and travel in a direction opposite to the incident radiation within a given solid angle.

One goal to be achieved is to be seen in providing a method for determining characteristics of inclusions in a simple way, avoiding the necessity of solving ill-posed mathematical problems and having to use complicated calibration procedures.

One way the goal is achieved is in providing a method for determining optical and spatial characteristics of an inclusion in a turbid medium using multiple-scattering optical tomography, comprising the steps of:
a) emitting a collimated laser incident radiation along an optical axis, originating from a continuous wave laser situated in a predefined position, into the turbid medium, whereby the incident radiation is scattered immediately after entering the turbid medium through a contact surface, the scattering yielding a totality of forward scattered primary photon beams comprised within a spread angle 8 and a totality of back-scattered secondary photon beams,
b) spatially filtering the totality of backscattered secondary photon beams, the filtering yielding a number of specific non-overlapped back scattered photon beams, wherein said specific photon beams have a substantially parallel orientation to the optical axis,
c) determining a spatial distribution of an optical output power of the turbid medium comprising the inclusion by measuring an optical output power of said specific back scattered photon beams,
d) computing the spatial characteristics of the inclusion based on the spatial distribution of the optical output power of the turbid medium comprising the inclusion, the said spatial characteristics comprising the lateral size and the three dimensional coordinates of the inclusion relative to a given reference position,
e) determining a contrast and a sign of the said contrast of the inclusion, based on a spatial distribution of an optical output power of the turbid medium without the inclusion and the spatial distribution of the optical output power of the turbid medium comprising the inclusion, and,
f) computing the optical characteristics of the inclusion and of the turbid medium based on the spatial characteristics and the spatial distribution of the optical output power of the turbid medium comprising the inclusion.

The turbid medium, like for example breast tissue, may contain inclusions, like for example a tumour. Several methods to detect the inclusion and determinate its type, like for example a malign or a benign tumour, are used, whereby a wide field is dedicated to employing high energetic X-Ray radiations. A main disadvantage of these methods is an unnecessary and potentially dangerous radiation, which may provoke unwanted alteration of the tissue.
The optical tomography uses light in order to determine certain characteristics of the tissue, in other words of the turbid medium, by exploiting the optical characteristics of the turbid medium and of the inclusion. The scattering angular dependence is a dependence on the angle between the direction of the incident radiation and the scattering direction and is sharply forward oriented meaning that it sharply drops to zero at an angle close to zero. A tissue-like turbid medium, like the breast tissue, is considered to be low absorbing and highly scattering in a range of visible and near-infrared light. The optical characteristics of the turbid medium and the inclusion are a basic criterion for differentiating the two media.
The spatial characteristics are: a position of the inclusion in the turbid medium relative to a fixed reference point, in other words a depth and lateral coordinates, and the dimensions of the turbid medium, thus providing information about a location, a lateral size and a lateral shape of the inclusion.
The incident radiation emitted by the laser is scattered immediately after entering the turbid medium, forming multiple photon beams comprised in the spread angle 8. The spread angle 8 depends only on the coordinate z and the optical characteristics of the turbid medium. It makes it possible to reach a better resolving quality of the inclusion.
A part of the forward scattered photon beams will eventually hit the inclusion. A portion of these photon beams is back-scattered and leave the turbid medium through the contact surface. Out of this portion of photon beams the specific, non-overlapped beams are filtered. By means of the substantially parallel beams, it is possible to measure the spatial distribution of the optical output power of the medium comprising the inclusion, the distribution being a basis for the computation of the spatial characteristics of the inclusion.
The spatial characteristics are detected for example by means of an optical detection and measurement system.
By measuring the spatial distribution of the medium in a position without the inclusion or with an inclusion, but in the position far from the inclusion and measuring the spatial distribution of the medium comprising the inclusion, the contrast and the sign of the contrast are determined by comparison of the two distributions.
The sign of the contrast is positive (positive inclusion) if the inclusion is optically denser than the medium and it is negative (negative inclusion) if the medium is optically denser than the inclusion.
Then, the optical characteristics of the inclusion and the turbid medium are computed based on the spatial characteristics and the spatial distribution of the optical output power.

The spatial filtering is accomplished by rejecting and/or by absorbing all particular backscattered photon beams which lack the substantial parallel orientation to the optical axis, out of the totality of backscattered photon beams, the rejection and/or the absorption being performed within at least one cylindrical aperture, the cylindrical aperture being parallel to the optical axis. The filtering is done by tiny cylinders which let pass only the specific photon beams with a substantially parallel orientation to the optical axis. This advantageously allows a detection of the distribution of the output power of backscattered beams which contain accurate information about a real position of the inclusion, whereas backscattered beams which are not substantially parallel to the incident radiation contain erroneous information about the real position of the inclusion.

According to a preferred method, multiple collimated laser incident radiations are emitted along parallel optical axes, each of the multiple radiations originating from a different continuous wave laser and being successively emitted one at a time, until all of the multiple radiations have been emitted. For each of the emitted multiple radiations a corresponding spatial distribution of the optical output power of the medium comprising the inclusion relative to a joint reference position is determined. This advantageously improves a precision of the method, especially in the respect of resolving the lateral size and the three dimensional coordinates.

A totality of the spatial distributions determined for each of the emitted multiple radiations are compared by software algorithms.

The spatial characteristics, the contrast and the sign of the said contrast of each one of multiple inclusions, and the optical characteristics of each one of multiple inclusions in the turbid medium are determined. A further advantage of the method is the possibility to determine multiple inclusions. Especially using an array of radiations, a wide area of the turbid medium is scanned.

A type of the turbid medium and a type of the inclusion is determined by comparing the optical characteristics with each characteristic of a set of reference optical characteristics of known types of turbid media and known types of inclusions. Different types of turbid media and different types of inclusions are determined in advance by measuring probes of a known turbid medium. By comparing a calculated or measured optical power distribution of reference probes with the optical power distribution of the turbid medium comprising the inclusion a nature of the turbid medium and of the inclusion is determined.

Features and advantages of the present invention will become more apparent from the following detailed description in conjunction with the following figures, whereby:
- Figure 1: a schematic view on a set-up with one inclusion, implementing the inventive method;
- Figure 2: a graph of experimentally measured backscattered optical output power distribution;
- Figure 3: a) an example of an experimentally measured optical power in the presence of a positive inclusion as a function of the inclusion depth, wherein the level of the optical power from the free medium (without any inclusion) is also marked and b) an example of an experimentally measured optical power in the presence of a negative inclusion as a function of the inclusion depth wherein the level of the optical power from the free medium (without any inclusion) is also marked;
- Figure 4: an example of simulated backscattered optical output power distributions;
- Figure 5: an example of a set-up with two inclusions, implementing the inventive method;
- Figure 6: a diagram of the inclusion signals for the set up as shown in figure 5;
- Figure 7: an example of resolving two inclusions in a two dimensional space; and
- Figure 8: a diagram of an estimated inclusion depth as a function of a true inclusion depth

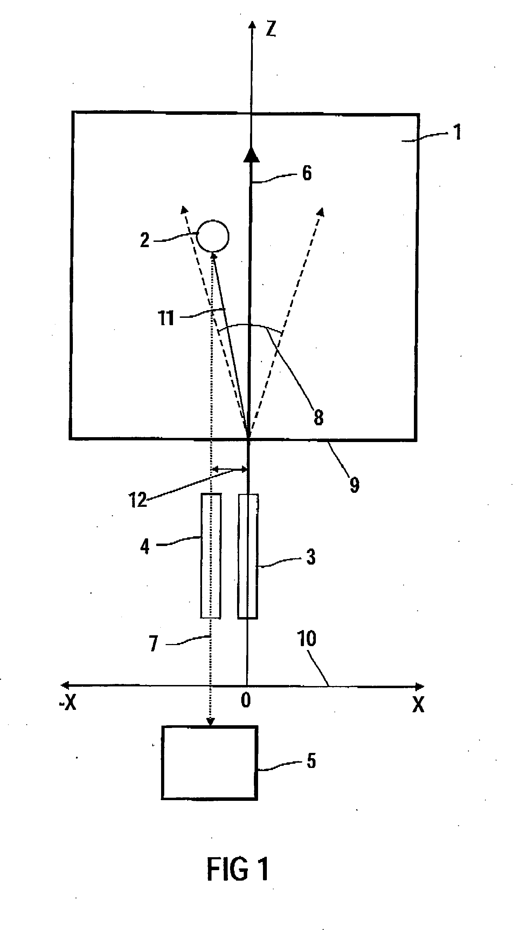

Figure 1 shows a two dimensional representation of the method, whereby an inclusion 2 is situated inside of a turbid medium 1. A laser 3 emits an incident radiation 6 in z-direction of a coordinate system 10. As soon as a contact surface 9 has been reached by the radiation 6, the radiation 6 is scattered in a spread angle 8. A forward scattered photon beam 11 hits the inclusion 2 and generates a back scattered photon beam 7.
Because of clarity reasons, only the photon beam 7, which is relevant for the description, is represented and for explanation purposes it is assumed that the photon beam 7 travels on an axis of the cylindrical aperture. Furthermore, also for clarity reasons, only a two dimensional representation is shown, although the method is equally valid for three dimensional determinations. Preferably the inclusion 2 has a spherical shape.
The photon beam 7, which is parallel to the incident radiation 6, passes through a cylindrical aperture 4 and strikes an optical detection and measurement system 5. A distance between the incident radiation 6 and the backscattered photon beam 7 is denoted as 12.

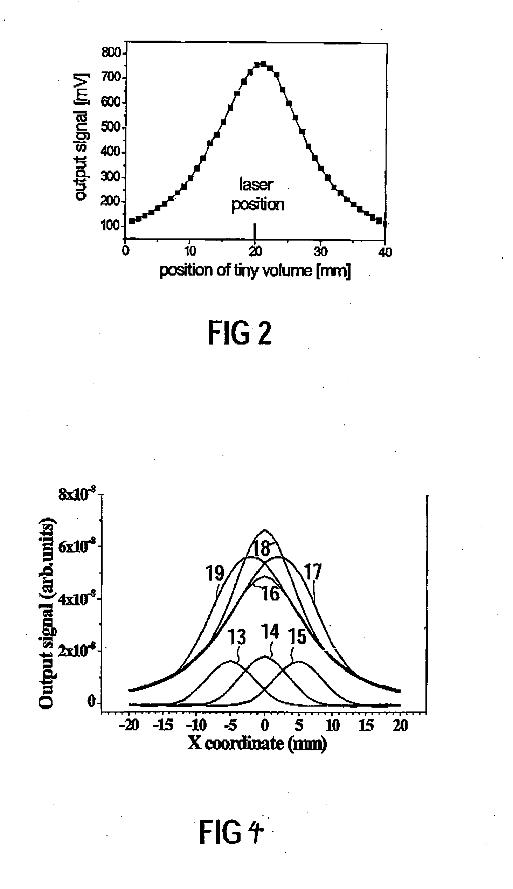

Figure 2 shows a diagram of experimentally measured backscattered optical output power distribution, denoted as a vertical axis of the diagram, as a function of a distance 12 between an optical axis of the cylindrical aperture 4, denoted as a horizontal axis of the diagram, and the incident radiation 6. This diagram is an example for a turbid medium without inclusion, as measured for the probe of a turbid medium.

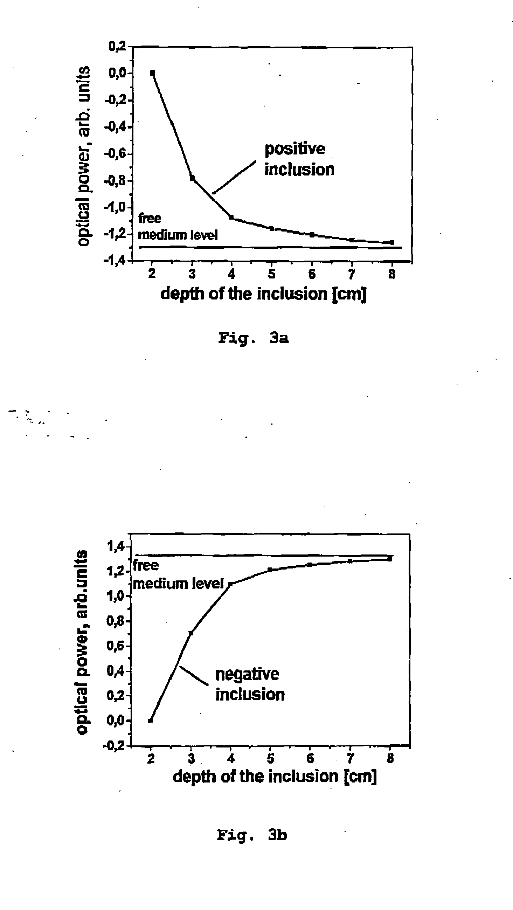

Figure 3a shows a diagram of experimentally measured back-scattered optical output power in a presence of a positive inclusion as a function of the inclusion depth. The level of the optical power from the free medium (without any inclusion) is also marked. The output power decreases with increasing the depth, but it always remains higher than the backscattered optical output power of a free medium. It is important to note that these changes of the optical power in the presence of an inclusion can be directly measured to large depths to the order of or higher than 8 cm. This diagram demonstrates the method capabilities to meet the requirements for optical tomography of breast tissues, etc.

Figure 3b shows a diagram of experimentally measured back-scattered optical output power in a presence of a negative inclusion as a function of the inclusion depth. The level of the optical power from the free medium (without any inclusion) is also marked. The behaviour of this dependence is opposite to the case in Figure 3a. Here the output power is increased with a depth increase, but it always remains below the backscattered optical output power of a free medium. It is important to note that these changes of the optical power can be directly measured to large depths to the order of or higher than 8 cm. This diagram demonstrates the method capabilities to meet the requirements for optical tomography of breast tissues, etc.

Figure 4 shows a diagram of simulated back-scattered optical output power distributions, denoted as a vertical axis of the diagram, as a function of a distance between the inclusion and the incident radiation. A first distribution 16 is recorded for the medium without the inclusion. A second distribution 18 is recorded for the medium with the inclusion situated directly on the z-axis, thus at x = 0. The difference to the first distribution 16 is that the output power is higher (positive inclusion), as in addition to backscattered photon beams of the turbid medium also backscattered photon beams of the inclusion are overlaid. A third and a fourth distribution 19 and 17 are recorded for the medium containing an inclusion at a position x = -5mm and at another position of x = 5mm respectively. The distributions 14, 13 and 15 represent only signals of the inclusion situated in the three previously mentioned positions respectively. The inclusion causes a shift of a maximum value of the distribution and thus an asymmetry when it is situated left or right of the incident radiation 6. The widths of the signals in the distributions 14, 13 and 15 are equal to a diameter of the inclusion.

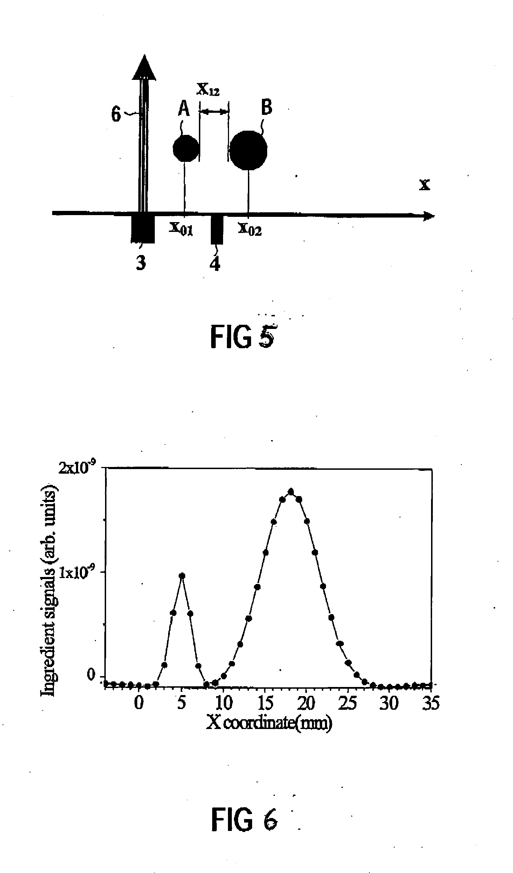

Figure 5 shows a disposition of two positive inclusions A and B in the turbid medium at the positions x01 and x02 respectively. The incident radiation 6 is emitted by the laser 3 in z-direction. The backscattered photon beams are filtered by the cylindrical aperture 4. A distance between the inclusions A and B is x12.

Figure 6 shows signals from the inclusions A and B from the figure 4 as a function of the distance from the laser. The inclusion A is detected at the position x = X01 = 5mm and the inclusion B is detected at the position x = x02 = 18mm. The first peak from the left in the output power is smaller because the inclusion A is smaller.

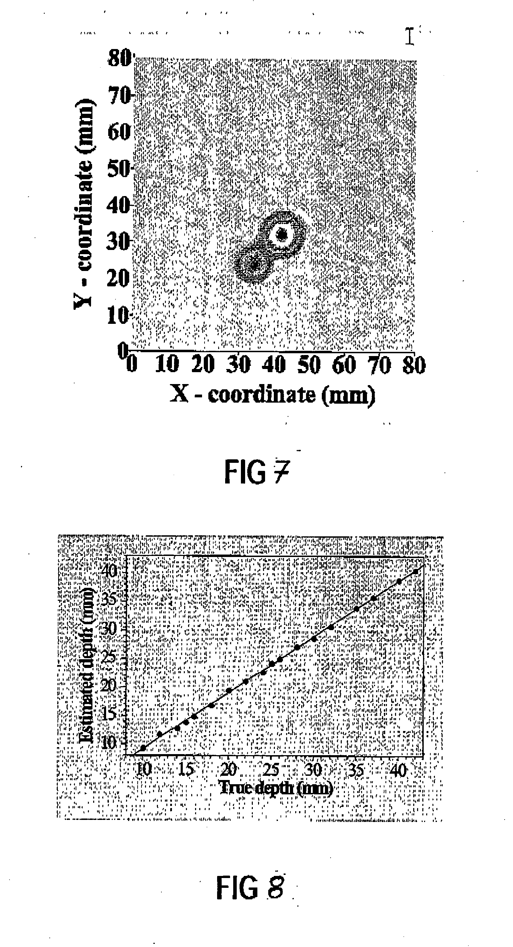

Figure 7 shows an example of two inclusions resolved in a two dimensional space. (*The circles are lines of equal optical powers.)*

Figure 8 illustrates the precision of the method, whereby a set of simulations are carried out with an inclusion situated at several known depths in the turbid medium. The estimated depths calculated by the method are shown as a function of the known depths.

### List of Abbreviations

- 0 =: origin of coordinate system
- 1 =: turbid medium
- 2 =: inclusion
- 3 =: laser
- 4 =: cylindrical aperture
- 5 =: optical detection and measurement system
- 6 =: incident radiation
- 7 =: back scattered photon beam
- 8 =: spread angle
- 9 =: contact surface
- 10 =: x-axis
- 11 =: forward scattered photon beam
- 12 =: distance between optical axis of cylindrical aperture and incident radiation
- 13 - 18 =: optical output power distributions
- A =: first inclusion
- B =: second inclusion
- x01 =: position of first inclusion
- x02 =: position of second inclusion
- x12 =: distance between inclusions

## Claims

1. Method for determining optical and spatial characteristics of an inclusion (2) in a turbid medium (1) using multiple-scattering optical tomography, comprising the steps of:
a) emitting a collimated laser incident radiation (6) along an optical axis (z), originating from a continuous wave laser (3) situated in a predefined position, into the turbid medium (1), whereby the incident radiation (6) is scattered immediately after entering the turbid medium (1) through a contact surface (9), the scattering yielding a totality of forward scattered primary photon beams comprised within a spread angle (8) and a totality of backscattered secondary photon beams,
b) spatially filtering the totality of backscattered secondary photon beams, the filtering yielding a number of specific non-overlapped back scattered photon beams, wherein said specific photon beams have a substantially parallel orientation to the optical axis (z),
c) determining a spatial distribution of an optical output power of the turbid medium (1) comprising the inclusion (2) by measuring an optical output power of said specific back scattered photon beams,
d) computing the spatial characteristics of the inclusion (2) based on the spatial distribution of the optical output power of the turbid medium (1) comprising the inclusion (2), the said spatial characteristics comprising the lateral size and the tree dimensional coordinates of the inclusion (2) relative to a given reference position,
e) determining a contrast and a sign of the said contrast of the inclusion, based on a spatial distribution of an optical output power of the turbid medium (1) without the inclusion (2) and the spatial distribution of the optical output power of the turbid medium (1) comprising the inclusion (2), and,
f) computing the optical characteristics of the inclusion (2) and of the turbid medium (1) based on the spatial characteristics and the spatial distribution of the optical output power of the turbid medium (1) comprising the inclusion (2).

2. Method according to claim 1, whereby the spatial filtering is accomplished by rejecting and/or by absorbing all particular backscattered photon beams which lack the substantial parallel orientation to the optical axis (z), out of the totality of backscattered photon beams, the rejection and/or the absorption being performed within at least one cylindrical aperture (4), the cylindrical aperture (4) being parallel to the optical axis (z).

3. Method according to claim 1 or 2, whereby multiple collimated laser incident radiations are emitted along parallel optical axes, each of the multiple radiations originating from a different continuous wave laser and being successively emitted one at a time, until all of the multiple radiations have been emitted.

4. Method according to claim 3, whereby for each of the emitted multiple radiations a corresponding spatial distribution of the optical output power of the medium comprising the inclusion relative to a joint reference position is determined.

5. Method according to claim 4, whereby a totality of the spatial distributions determined for each of the emitted multiple radiations are compared by software algorithms.

6. Method according to one of the preceding claims, whereby the spatial characteristics, the contrast and the sign of the said contrast of each one of multiple inclusions, and the optical characteristics of each one of multiple inclusions in the turbid medium (1) are determined.

7. Method according to one of the preceding claims, whereby a type of the turbid medium (1) and a type of the inclusion (2) is determined by comparing the optical characteristics with each characteristic of a set of reference optical characteristics of known types of turbid media and known types of inclusions.
